# EUROPEAN PATENT APPLICATION

(11) **EP 1 125 584 A1**
(43) Date of publication of application: **22.08.2001**
(21) Application number: 99951127.2
(22) Date of filing: 28.10.1999
(51) Int. Cl.: A61K 38/00, A61K 38/08, A61K 38/10, A61K 38/17, A61P 3/10

(54) **BETACELLULIN PROTEIN-CONTAINING PREPARATIONS**

(30) Priority: 30.10.1998 JP 31034398
(71) Applicant: Takeda Chemical Industries, Ltd., Osaka-shi, Osaka 541-0045 (JP)
(72) Inventor: SATO, Jun, Kawanishi-shi, Hyogo 666-0143 (JP); OMACHI, Yoshihiro, Osaka-shi, Osaka 432-0023 (JP)
(74) Representative: Caffin, Lee
(86) International application number: JP9905989
(87) International publication number: WO0025803

(57) **Abstract**

1) A sustained release preparation comprising a betacellin protein or a mutein thereof or a salt thereof, and 2) an agent for local administration to pancreas comprising a betacellin protein or a mutein thereof or a salt thereof, of the present invention, improve pancreatic function and are useful for treating or preventing diabetes and the like.

## Description

### TECHNICAL FIELD

The present invention relates to a preparation comprising a betacellulin protein or a mutein thereof or a salt thereof.

### BACKGROUND ART

Betacellulin protein (hereinafter sometimes abbreviated as BTC protein) is a proteinous factor produced by pancreatic beta tumor cells derived from transgenic mice, and its entire amino acid sequence is revealed by the cDNA analysis (Shing et al.; Science, 259: 1604 (1993), Sasada et al.; Biochemical Biophysical Research Communications, 190: 1173 (1993)).

Initially, BTC protein was found to be a factor having proliferation promoting activity against mouse 3T3 cells and, thereafter, found to have proliferation promoting activity also against vascular smooth muscle cells and retinal pigmented epithelial cells (Shing et al.; Science, 259:1604 (1993)). In addition, it has been reported that BTC protein can be used for making a competing agent (for example, antibody, pseudopeptide and the like) which can be employed for treating wound, ulcer or vascular abnormality, for treating diseases caused by smooth muscle cell proliferation such as atherosclerosis and diabetic retinopathy recognized in diabetes (JP-A 4-352800 and JP-A 6-87894).

On the other hand, diabetes is known to be a disease caused by a variety of complications due to prolonged hyperglycemia. This disease is classified into an insulin-dependent type, a non insulin-dependent type and others, and is considered to develop by a variety of causes. However, the disease develops basically due to lack of insulin action and, particularly in an insulin-dependent type diabetes, its main cause is an absolute lack of produced insulin.

Kojima et al. report that, when BTC protein having an activity of promoting differentiation of undifferentiated pancreatic stem cells into pancreatic beta cells producing insulin was subcutaneously administered to alloxan pancreatic part perfusion diabetic model mice at 1 mg/kg weight/day every day for 8 weeks, glucose tolerance ability was improved, and that a pancreatic function improving agent comprising BTC protein or a mutein thereof is useful for treating or preventing diabetes and the like (JP-A 9-188630 and the like).

Since intracortoral kinetics of BTC protein or a mutein thereof or a salt thereof has a large distribution volume and intracortoral disappearance clearance, and since the blood half life is about 20 minutes, BTC protein or a mutein thereof or a salt thereof needs to be administered at a higher dose for a long period of time in order to obtain a pancreatic function improving effect. However, for example, when an aqueous solution of BTC protein or a mutein thereof or a salt thereof is administered subcutaneously, the manifestation of side effects and inconvenience and pain of a patient in actual administration for treatment or prevention are matters of concern. Accordingly, there is desired development of useful preparations comprising BTC protein or a mutein thereof or a salt thereof which can solve these problems,

### DISCLOSURE OF INVENTION

In view of the above problems, the present inventors made extensive investigations, and, as a result, found that, when BTC protein or a mutein thereof or a salt thereof is administered (particularly locally administered) in the form of a sustained release preparation, it unexpectedly acts on pancreatic exocrine gland-derived cells having pluripotency and makes those cells differentiate effectively into beta cells, and found that since such BTC protein or a mutein thereof or a salt thereof is sustained released over a long period of time and a higher dose is not required to obtain the drug efficacy, reduction of side effects is attained, and that since successive administration is not necessary, inconvenience and pain of a patient are alleviated and, thus, the present preparation has excellent properties as a clinical medicine.

Further, the present inventors found that pancreatic function is effectively improved by administering a preparation comprising BTC protein or a mutein thereof or a salt thereof locally to the pancreas, which resulted in completion of the present invention.

That is, the present invention provides:
(1) a sustained release preparation comprising a betacellulin protein or a mutein thereof or a salt thereof,
(2) the sustained release preparation according to the above (1), wherein the betacellulin protein is a protein having the amino acid sequence represented by at least one SEQ ID No. selected from the group consisting of SEQ ID No.:3, SEQ ID No.:4, SEQ ID No.:5 and SEQ ID No.:6,
(3) the sustained release preparation according to the above (1), wherein the betacellulin protein is a protein having the amino acid sequence represented by SEQ ID No. :1 or a protein having the amino acid sequence represented by SEQ ID No.:2,
(4) the sustained release preparation according to the above (1), wherein the betacellulin protein is a protein having the amino acid sequence represented by SEQ ID No.:1,
(5) the sustained release preparation according to the above (1), wherein the mutein of a betacellulin protein is a protein having ① the amino acid sequence in which about 1 to 40 amino acids of the amino acid sequence represented by SEQ ID No.:1 or SEQ ID No.:2 are missing, ② the amino acid sequence in which about 1 to 40 amino acids of the amino acid sequence represented by SEQ ID No.:1 or SEQ ID No.:2 are replaced by other amino acids, or ③ the amino acid sequence in which about 1 to 40 amino acids are added to the amino acid sequence represented by SEQ ID No.:1 or SEQ ID No.:2,
(6) the sustained release preparation according to the above (1), wherein the mutein of a betacellulin protein is a protein having the amino acid sequence in which 12 or 30 amino acids are missing from an N terminal of the amino acid sequence represented by SEQ ID No.:1,
(7) the sustained release preparation according to the above (1), which comprises a betacellulin protein or a mutein thereof or a salt thereof and a carrier,
(8) the sustained release preparation according to the above (7), wherein the carrier is a polymer,
(9) the sustained release preparation according to the above (8), wherein the polymer is a biodegradable polymer,
(10) the sustained release preparation according to the above (9), wherein the biodegradable polymer is an aliphatic polyester,
(11) the sustained release preparation according to the above (10), wherein the aliphatic polyester is lactic acid-glycolic acid polymer,
(12) the sustained release preparation according to the above (11), wherein the lactic acid/glycolic acid composition ratio of the lactic acid-gycolic acid polymer is about 100/0 to about 40/60,
(13) the sustained release preparation according to the above (11), wherein a weight average molecular weight of the lactic acid-glycolic acid polymer is about 3,000 to about 80,000,
(14) the sustained release preparation according to the above (8), wherein the polymer is a non-biodegradable polymer,
(15) the sustained release preparation according to the above (14), wherein the non-biodegradable polymer is a polyglycerin fatty acid ester,
(16) the sustained release preparation according to the above (1), which is a pancreatic function improving agent,
(17) the sustained release preparation according to the above (1), which is an agent for treating or preventing diabetes,
(18) the sustained release preparation according to the above (1), which is an agent for parenteral administration,
(19) the sustained release preparation according to the above (1), which is an agent for local administration to the pancreas,
(20) the sustained release preparation according to the above (1), which is an agent for subcutaneous administration,
(21) the sustained release preparation according to the above (1), which is an agent for intramuscular administration,
(22) the sustained release preparation according to the above (1), which is an agent for intraperitoneal administration,
(23) an agent for local administration to the pancreas, which comprises a betacellulin protein or a mutein thereof or a salt thereof,
(24) use of a betacellulin protein or a mutein thereof or a salt thereof for the manufacture of a sustained release preparation,
(25) use of a betacellulin protein or a mutein thereof or a salt thereof for the manufacture of an agent for local administration to the pancreas,
(26) a method for treating diabetes, which comprises administering an effective amount of a sustained release preparation as defined in the above (1) to a mammal, and
(27) a method for treating diabetes, which comprises locally administering an effective amount of an agent for local administration as defined in the above (23) to a mammal.

### BRIEF DESCRIPTION OF THE DRAWINGS

Fig.1 is a graph showing changes with time in the remaining rhBTC(%) after subcutaneous administration of sustained release PLGA microcapsules comprising rhBTC in rats. ● denotes a dose of 3.2 mg/kg, ○ denotes a dose of 5.9 mg/kg, and ▲ denotes a dose of 10.0 mg/kg.

Fig.2 is a graph showing changes with time in blood rhBTC concentration (pg/ml) after subcutaneous administration of sustained release PLGA microcapsules comprising rhBTC in rats. ● denotes a dose of 3.2 mg/kg, ○ denotes a dose of 5.9 mg/kg, and ▲ denotes a dose of 10.0 mg/kg.

Fig.3 is a graph showing changes with time in the remaining rhBTC(%) after subcutaneous administration of a sustained release HGPS preparation comprising rhBTC in a rat. Dose denotes 4.0 mg/kg.

Fig.4 is a graph showing the blood glucose concentration (mg/dl) 8 weeks after administration of a sustained release HGPS preparation comprising rhBTC in STZ diabetes model rats. ● denotes a non-treated diabetes rat group, ○ denotes a normal rat group, ▼ denotes a pancreatic local administration group (dose 45 mg/kg), and ∇ denotes a subcutaneous administration group (dose mg/kg).

Fig. 5 is a graph showing changes with time in the blood insulin concentration (µU/ml) 8 weeks after administration of a sustained release HGPS preparation comprising rhBTC in STZ diabetes model rats. ● denotes a non-treated diabetes rat group, ○ denotes a normal rat group, ▼ denotes a pancreatic local administration group (dose 45 mg/kg), and ∇ denotes a subcutaneous administration group (dose 45 mg/kg).

### BEST MODE FOR CARRYING OUT THE INVENTION

A BTC protein or a mutein thereof employed in the present invention may be any protein as long as it has a BTC-like activity, that is, the activity of promoting proliferation of cells such as fibroblast, vascular smooth muscle cell, retinal pigmented epithelial cells and the like, more particularly, the activity of promoting differentiation from undifferentiated pancreatic stem cells to pancreatic beta cells. In addition, for example, fermentation products, synthetic compounds, synthetic peptides and the like can be employed.

Alternatively, BTC protein may be a naturally derived protein or a recombinant protein produced by a genetic engineering method.

As a naturally derived BTC protein, for example, BTC proteins derived from any mammal such as human beings, monkeys, sacred baboons, chimpanzees, pigs, cows, sheep, horses, mice, rats and the like are employed. Among these, BTC protein derived from human beings and mice is preferable , and BTC protein derived from human beings is particularly preferable. When a preparation of the present invention is administered to human beings, use of BTC protein derived from human beings is particularly preferable.

As BTC protein, specifically, a protein having an amino acid sequence represented by at least one sequence number selected from the group consisting of SEQ ID No.:3, SEQ ID No.:4, SEQ ID No.:5 and SEQ ID No.:6 is employed. More specifically, as BTC protein derived from human beings, for example, a protein having an amino acid sequence represented by SEQ ID No.:1 (JP-A 6-87894) is employed. As BTC protein derived from mice, for example, a protein having an amino acid sequence represented by SEQ ID No.:2 (Shing et al; Science, 259:1604 (1993)) is employed.

In addition, these BTC proteins may be a simple protein composed of only amino acids or a conjugated protein such as glycoprotein, lipoprotein, hemeprotein, metalloprotein, flavoprotein, phosphoprotein and the like. In the case of a glycoprotein, as a sugar chain, there are neutral sugars such as D-mannose, D-galactose, L-fructose and the like, amino sugars such as D-glucosamine, D-glactosamine and the like, and sialic acid and the like.

As a mutein of BTC protein, for example, a missing type mutein in which at least one constituent amino acid of the above BTC protein is missing, a substituted type mutein in which at least one constituent amino acid of BTC protein is replaced by another amino acid, an addition type mutein in which at least one amino acid is added to BTC protein and the like are used. The number of missing, substituted or added amino acids may be any number as long as the inherent activity of BTC protein is not lost.

Specifically, employed is a protein having ① an amino acid sequence in which about 1 to 40 amino acids of an amino acid sequence represented by SEQ ID No.:1 or SEQ ID No.:2 are missing, ② an amino acid sequence in which about 1 to 40, preferably about 1 to 9 amino acids of an amino acid sequence represented by SEQ ID No.:1 or SEQ ID No.2 are replaced by other amino acid sequence, or ③ an amino acid sequence in which about 1 to 40, preferably about 1 to 9 amino acids are added to an amino acid sequence represented by SEQ ID No.:1 or SEQ ID No.:2. Among these, preferred is a protein having at least one amino acid sequence selected from the group consisting of the following partial amino acid sequences:
① His Phe Ser Arg Cys Pro Lys Gln Tyr Lys His Tyr Cys Ile (SEQ ID No.:3)
② Gly Arg Cys Arg Phe Val Val (SEQ ID No.:4)
③ Glu Gln Thr Pro Ser Cys (SEQ ID No.:5) and
④ Gly Ala Arg Cys Glu Arg Val Asp Leu Phe Tyr (SEQ ID No.:6).

Among these muteins, a missing type mutein and the like are preferable and, for example, preferred is a protein having an amino acid sequence in which about 1 to 40 amino acids are missing from an N-terminal of an amino acid sequence represented by SEQ ID No.:1 or SEQ ID No.:2, and the like.

More specifically, as a missing type mutein of BTC protein, for example, a missing type mutein of human BTC protein having an amino acid sequence in which 12 or 30 amino acids are missing from an N-terminal of an amino acid sequence represented by SEQ ID No.: 1 or SEQ ID No.: 2, and the like are employed (Watanabe et al.; Journal of Biochemistry, 269: 9966 (1994)). In particular, preferred is a missing type mutein of human BTC protein having an amino acid sequence in which 12 or 30 amino acids are missing from an N-terminal of an amino acid sequence represented by SEQ ID No.:1.

As the other mutein of BTC protein, any one may be employed as long as the activity of BTC protein is not lost, and it includes a mutein in which an N-terminal of BTC protein is acylated (e.g., C₁₋₆ acyl such as C₂₋₆ alkanoyl such as formyl and acetyl), glycosylated, or chemically modified (such as polyethylene glycol derivative and like).

In addition, in the BTC protein or mutein thereof, a carboxyl group of a C-terminal may be amidated or esterified (e.g., C₁₋₆ alkyl-ester such as methyl, ethyl, n-propyl, isopropyl, n-butyl and the like).

A BTC protein or mutein thereof may form a salt. As a salt of BTC protein or a mutein thereof, pharmacologically acceptable salts are especially preferable. As such salts, for example, employed are salts with an inorganic acid (e.g., hydrochloric acid, phosphoric acid, hydrobromic acid, sulfuric acid and the like), salts with an organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid and the like), salts with an inorganic base (e.g., alkali metal salts such as sodium salts, potassium salts and the like; alkaline earth metal salts such as calcium salts, magnesium salts and the like; aluminium salts, ammonium salts and the like), salts with an organic base (e.g., trimethylamine, triethylamine, pyridine, picoline, ethanolamine, diethanolamine, triethanolamine, dicyclohexylamine, N,N-dibenzylethylenediamine and the like).

Among BTC proteins or muteins thereof employed in the present invention, a human BTC protein having an amino acid sequence represented by SEQ ID No.:1, a missing type human BTC mutein in which 12 or 30 amino acids are missing from an N-terminal of an amino acid sequence represented by SEQ ID No.:1, and a mouse BTC protein having an amino acid sequence represented by SEQ ID No.:2 are known. In addition, other BTC proteins or muteins thereof can be prepared by per se known methods or analogous methods thereto. For example, they can be prepared by peptide synthesizing methods known per se, or by cutting known peptides or precursors with a suitable peptitase. The peptide synthesizing method may be, for example, any of a solid phase synthesizing method and a liquid phase synthesizing method. That is, the desired peptide can be prepared by condensing a partial peptide or an amino acid which constitutes the peptide or a precursor, with the remaining part and, when a product has a protecting group, leaving the protecting group. As the known condensing method or protecting group leaving method, there are methods described in the following ① to ⑤:
① M.Bodanszky and M. A. Ondetti, Peptide Synthesis, Interscience Publishers, New York (1996),
② Schroeder and Luebke, The peptide, Academic Press, New York (1965),
③ Nobuo Izumiya et al, Base and Experiment on Peptide Synthesis, Maruzen (K.K.) (1975),
④ Haruaki Yajima and Shunpei Sakakibara, Biochemistry Experimental Course 1, Chemistry of Peptide or Precursor IV, 205, (1977),
⑤ supervised by Haruaki Yajima, Development of Drugs, a Second Series, vol. 14, Peptide Synthesis, Hirokawa Shoten.

In addition, after the reaction, the desired peptide or precursor can be purified and isolated by combining the normal purifying methods such as solvent extraction, distillation, column chromatography, liquid chromatography, recrystallization and the like. When the peptide or precursor obtained by the above method is a free form, it can be converted into a suitable salt by a known method and, conversely, when obtained as a salt, it can be converted into a free form by a known method.

A sustained release preparation comprising BTC protein or a mutein thereof or a salt thereof of the present invention preferably comprises a carrier (a carrier or a base) and examples of the carrier include a protein, polysaccharide, a polymer (a biodegradable polymer, a non-biodegradable polymer), a liposome, an inorganic substance and the like.

Examples of the protein include collagen, gelatin, fibrin, serum albumin, and the like.

Examples of the polysaccharide include starch, hyaluronic acid, chitosan, chitin, alginate, agarose, dextran, a cellulose derivative and the like.

Examples of the biodegradable polymer include an aliphatic polyester, polophophabenzene, polyorthoester and the like.

Examples of the non-biodegradable polymer include a polyglycerin fatty acid ester, silicone, an ethyl vinyl acetate copolymer, a polyhydroxyethylmethyl methacrylate, a polyvinyl alcohol, a polypyrrolidone and the like.

Examples of the liposome include a polysaccharide-modified liposome and the like in addition to a normal liposome.

Examples of the inorganic substance include hydroxyapatite, a tricalcium phosphate, a calcium carbonate, a calcium sulfate and the like.

As the carrier employed in a sustained release preparation of the present invention, for example, a polymer (such as a biodegradable polymer, a non-biodegradable polymer and the like) and the like are preferable. In particular, as a biodegradable polymer, an aliphatic polyester and the like are preferable and, as a non-biodegradable polymer, a polyglycerin fatty acid ester and the like are preferable.

As the aliphatic polyester, for example, a lactic acid-glycolic acid polymer and the like are used.

The composition ratio of the lactic acid-gycolic acid polymer (lactic acid/glycolic acid; L/G ratio) (mol%) is preferably about 100/0 to 40/60, more preferably about 100/0 to about 75/25, particularly preferably 100/0 (polylactic acid).

The weight average molecular weight of the lactic acid-glycolic acid polymer is preferably about 3,000 to about 80,000, more preferably about 5,000 to about 25,000, particularly preferably about 7,000 to about 20,000.

The degree of dispersion (weight average molecular weight/number average molecular weight) of the lactic acid-glycolic acid polymer is preferably about 1.2 to about 4.0, more preferably about 1.5 to about 3.5.

Examples of the polyglycerin fatty acid ester include tetraglycerol monopalmitate (TGMP), tetraglycerol dipalmitate (TGDP), tetraglycerol tripalmitate (TGTP), tetraglycerol hexapalmitate (TGHP), tetraglycerol monostearate (TGMS), tetraglycerol distearate (TGDS), tetraglycerol tristearate (TGTS), tetraglycerol hexastearate (TGHS), hexaglycerol pentastearate (HGPS), tetraglycerol hexapalmitate (TGHP) and the like. These may be used alone or as a mixture thereof.

A sustained release preparation of the present invention can be prepared by per se known methods.

Specifically, a sustained release preparation of the present invention can be prepared by mixing BTC protein or a mutein thereof or a salt thereof with a carrier normally employed in preparing a sustained release preparation (for example, the aforementioned carrier) and, if necessary, molding it. For example, the carrier is used so that the amount of BTC protein or a mutein thereof or a salt thereof used is about 0.01 to about 50 %(w/w) relative to the carrier.

As the sustained release preparation of the present invention, a method for producing A) a sustained release preparation comprising BTC protein using as a carrier a polyglycerin fatty acid ester which is a non-biodegradable polymer, and B) a sustained release preparation comprising BTC protein using as a carrier a lactic acid-glycolic acid polymer which is a biodegradable will be exemplified below.

### A) A sustained release preparation comprising BTC protein using a polyglycerin fatty acid ester as a carrier

Polyglycerin fatty acid ester is warmed until it melts, and a powder of BTC protein or a mutein thereof or a salt thereof is added, which is uniformly dispersed by stirring or the like. The dispersion is cooled and molded into a disc, a film, a bar or the like. Thereby, a sustained release preparation of polyglycerin fatty acid ester comprising a predetermined amount of BTC protein or a mutein thereof or a salt thereof can be prepared. A warming temperature is about 50 to about 100°C and a cooling temperature is about 0 to about 40°C. The amount of BTC protein or a mutein thereof or a salt thereof to be used differs depending upon the kind of BTC protein or mutein thereof or salt thereof, the desired pharmacological effect and duration time of the effect, and is about 0.1 to about 50% (w/w) relative to polyglycerin fatty acid ester.

### B) A sustained release preparation comprising BTC protein using a lactic acid-glycolic polymer as a carrier

### B-1) A method for producing a molded bar and the like will be described in detail.

### B-1-a)

A lactic acid-glycolic acid polymer is dissolved with an organic solvent (preferably, dichloromethane and the like), an aqueous solution of BTC protein or a mutein thereof or a salt thereof is added and the mixture is emulsified. This is dried in vacuum to obtain powders of a lactic acid-glycolic acid polymer in which BTC protein or a mutein thereof or a salt thereof is uniformly dispersed. This is warmed and cooled to mold into a disc, a film, a bar (a rod) or the like. Thereby, a sustained release preparation of a lactic acid-glycolic acid polymer comprising a predetermined amount of BTC protein or a mutein thereof or a salt thereof can be obtained. The warming temperature is about 50 to about 100°C, and the cooling temperature is about 0 to about 40°C. The amount of BTC protein or a mutein thereof or a salt thereof to be used differs depending upon the kind of BTC protein or mutein thereof or salt thereof, the desired pharmacological effect and the duration time of the effect, and is about 0.1 to about 30% (w/w) relative to a lactic acid-glycolic acid polymer.

### B-1-b)

A lactic acid-glycolic acid polymer is dissolved with an organic solvent (preferably, dichloromethane and the like), and powders of BTC protein or a mutein thereof or a salt thereof are added, which is uniformly dispersed. The resulting dispersion system is dried under vacuum to obtain powders of a lactic acid-glycolic acid polymer in which BTC protein or a mutein thereof or a salt thereof is uniformly dispersed. This is warmed and cooled to mold into a disc, a film, a bar (a rod) or the like. Thus, a sustained release preparation of a lactic acid-glycolic acid polymer comprising a predetermined amount of BTC protein or a mutein thereof or a salt thereof can be obtained. The warming temperature, the cooling temperature, and the amount of BTC protein or a mutein thereof or a salt thereof to be used are as described above.

### B-2) A method for producing microcapsules (also referred to as microspheres) will be described in detail.

A W/O/W emulsion and an O/W emulsion can be prepared respectively by (i) obtaining a W/O emulsion having an aqueous solution, a dispersion or a suspension of BTC protein or a mutein thereof or a salt thereof as an inner aqueous phase and a solution of a lactic acid-glycolic acid polymer in an organic solvent as an oily phase, or (ii) dissolving or suspending BTC protein or a mutein thereof or a salt thereof in a solution of a lactic acid-glycolic acid polymer in an organic solvent to obtain an oily phase, and adding this (i) or (ii) to water (external aqueous phase), followed by dispersion and emulsification.

The above (i), namely a W/O emulsion having an aqueous solution, a dispersion or a suspension of BTC protein or a mutein thereof or a salt thereof as an inner aqueous phase and a solution of a lactic-glycolic acid polymer in an organic solvent as an oily phase can be produced as follows.

First, BTC protein or a mutein thereof or a salt thereof is dissolved, dispersed or suspended in water to produce an inner aqueous phase. The concentration of the BTC protein or a mutein thereof or a salt thereof in the aqueous solution, dispersion or suspension is, for example, 0.001 to 90% (w/w), preferably 0.01 to 80% (w/w).

The amount of the above BTC protein or a mutein thereof or a salt thereof to be used differs depending upon the kind of BTC protein or mutein thereof or salt thereof, the desired pharmacological effect and the duration time of the effect, and is about 0.01 to about 50% (w/w), preferably about 0.1 to about 40% (w/w), particularly preferably about 1 to about 30% (w/w).

If necessary, in order to increase entrapment of BTC protein or a mutein thereof or a salt thereof in microcapsules, a drug retaining substance such as gelatin, agar, sodium alginate, polyvinyl alcohol or a basic amino acid (e.g., arginine, histidine, lysine and the like) may be added to the inner aqueous phase. The amount of the drug retaining substance to be added is usually about 0.01 to about 10 weight-fold relative to BTC protein or a mutein thereof or a salt thereof.

After an inner aqueous phase is lyophilized to the powder state once, it may be dissolved by addition of water to an appropriate concentration.

Separately, a lactic acid-glycolic acid polymer is dissolved in an organic solvent to produce an oily phase.

Examples of the above organic solvent include halogenated hydrocarbons (e.g., dichloromethane, chloroform, chloroethane, trichloroethane, carbon tetrachloride and the like), fatty acid esters (e.g., ethyl acetate, butyl acetate and the like), aromatic hydrocarbons (e.g., benzene, toluene, xylene and the like). Among these, dichloromethane is preferable.

The concentration of a lactic acid-glycolic acid polymer in an organic solvent differs depending upon a kind and a molecular weight of the lactic acid-glycolic acid polymer and the kind of the organic solvent, and [weight of a lactic acid-glycolic acid polymer/(weight of an organic solvent + weight of a lactic acid-glycolic acid polymer)] (×100%) is usually about 0.01 to about 90% (w/w), preferably about 0.01 to about 70% (w/w). It is preferable to dissolve without remaining insolubles.

To the thus obtained solution of a lactic acid-glycolic acid polymer in an organic solvent (oily phase) is added the above mentioned aqueous solution, dispersion or a suspension of BTC protein or a mutein thereof or a salt thereof (inner aqueous phase), which is dispersed and emulsified with a homomixer or the like to prepare a W/O emulsion.

On the other hand, the above (ii), namely, an oily phase obtained by dissolving or suspending BTC protein or a mutein thereof or a salt thereof in a solution of a lactic acid-glycolic acid polymer in an organic solvent can be produced as follows.

First, a solution of a lactic acid-glycolic acid polymer in an organic solvent is produced. The same organic solvent as that used upon production of the above W/O emulsion can be used.

The concentration of a lactic acid-glycolic acid polymer in a solution of an organic solvent differs depending upon the molecular weight of the lactic acid-glycolic acid polymer and the kind of organic solvent, and [weight of a lactic acid-glycolic acid polymer/(weight of the organic solvent + weight of a lactic acid-glycolic acid polymer)](×100%) is usually about 0.01 to about 70% (w/w), preferably about 1 to about 60% (w/w).

Next, BTC protein or a mutein thereof or a salt thereof is dissolved or suspended in a solution of a lactic acid-glycolic acid polymer in an organic solvent to produce an oily phase.

The amount of BTC protein or a mutein thereof or a salt thereof to be used may be selected so that the ratio of the BTC protein or a mutein thereof or a salt thereof to a lactic acid-glycolic acid polymer is similar to that for producing the above W/O emulsion (i).

Then, the aforementioned (i) W/O emulsion or (ii) oily phase is added to an external aqueous phase, which is dispersed and emulsified with a homomixer or the like to produce a W/O/W emulsion or an O/W emulsion.

The amount of the external aqueous phase to be used is about 1 to about 10000 volume-fold, preferably about 10 to about 2000 volume-fold, particularly preferably about 50 to about 500 volume-fold relative to the above (i) or (ii).

An emulsifying agent is usually added to an external aqueous phase. Any emulsifying agent which can generally form a stable W/O/W emulsion or O/W emulsion may be used. Examples thereof include anionic surfactants, nonionic surfactants, polyoxyethylene castor oil derivatives, polyvinylpyrrolidone, polyvinyl alcohol, carboxymethyl cellulose, lecithin, gelatin, hyaluronic acid and the like. Among these, polyvinyl alcohol is preferable. The concentration of the emulsifying agent in an external aqueous phase is usually about 0.001 to about 20% (w/w), preferably about 0.01 to about 10% (w/w), particularly preferably about 0.05 to about 5% (w/w).

By subjecting the thus obtained W/O/W emulsion or O/W emulsion (hereinafter abbreviated as merely emulsion in some cases) to an in-water drying method, an organic solvent contained in these emulsions can be removed to produce microcapsules.

The thus obtained microcapsules are recovered by centrifugation or a sieve or the like and, if desired, anticoagulants such as sugar or sugar alcohol, inorganic salts and the like, preferably mannitol, sorbitol and the like are added in order to prevent aggregation of microcapsules, which are thereafter subjected to lyophilization.

A mixing ratio (weight ratio) of microcapsules and anticoagulants is about 50:1 to about 1:1, preferably about 20:1 to about 1:1, more preferably about 10:1 to about 5:1.

A method of adding the anticoagulants is not particularly limited so long as it is a method for mixing microcapsules and anticoagulants uniformly and, for example, there is a method of dispersing microcapsules in an aqueous solution of anticoagulants.

In a process for producing sustained release microcapsules of BTC protein or a mutein thereof or a salt thereof, microencapsulation by an in-water drying method is often preferable.

The thus obtained microcapsules are, if necessary, warmed to dry under reduced pressure to remove more completely water and a solvent in microcapsules.

In addition, besides a method using the aforementioned W/O/W emulsion or O/W emulsion, production may be performed by a S/O/W method which comprises removing a solvent from a S/O type dispersion in which powders of BTC protein or a mutein thereof or a salt thereof (S phase) are dispersed in a solution of a lactic acid-glycolic acid polymer in an organic solvent (O phase).

In the present method, first, a lactic-glycolic acid polymer is dissolved in an organic solvent and powders of BTC protein or a mutein thereof or a salt thereof (S phase) is added to and dispersed in this organic solvent solution. Here, a mixing ratio (weight ratio) of the BTC protein or a mutein thereof or a salt thereof and a lactic acid-glycolic acid polymer is, for example, about 1:1000 to about 1:1, preferably about 1:200 to 1:5, more preferably about 1:100 to about 1:5. In addition, it is preferable to add external physical energy in order to disperse uniformly powders of BTC protein or a mutein thereof or a salt thereof in an organic solvent. Examples of such methods include, for example, ultrasound irradiation, turbine type stirrer, homogenizer and the like.

Then, the thus prepared organic solvent dispersion (S/O type dispersion) is further added to an aqueous solvent (W phase) and a S/O/W type emulsion is formed by similar external physical energy to that described above, for example, ultrasonic irradiation, turbine stirrer, homogenizer or the like. Thereafter, an oily phase solvent is distilled out to produce microcapsules. Upon this, the volume of an aqueous phase is generally selected from about 1-fold to about 10,000-fold of an oily phase volume. The aqueous phase volume is more preferably selected from about 2-fold to about 5,000-fold, particularly preferably about 5-fold to about 2,000-fold.

An emulsifying agent may be added to the above external aqueous phase. As the emulsifying agent, any emulsifying agents may be generally used which can form a stable S/O/W emulsion. Examples of the emulsifying agent include anionic surfactants, nonionic surfactants, polyoxyethylene castor oil derivatives, polyvinylpyrrolidone, polyvinyl alcohol, carboxymethyl cellulose, lecithin, gelatin, hyaluronic acid and the like. These may be appropriately used in a combination. The concentration of the emulsifying agent in an external aqueous phase is preferably about 0.001% to about 20% (w/w). It is more preferably about 0.01% to about 10% (w/w), particularly preferably about 0.05% to about 5% (w/w).

The thus obtained microcapsules are collected by centrifugation or filtration procedures and, thereafter, an emulsifying agent and the like attached to the surface of microcapsules are removed by washing with distilled water and the microcapsules are dispersed again in distilled water or the like to lyophilize. Thereafter, if necessary, microcapsules are warmed to further remove water and an organic solvent in the microcapsules. It may be warmed under reduced pressure. As the warming condition, microcapsules are heated to dry at least at a glass transition temperature of a lactic acid-glycolic acid polymer used, and at a temperature at which respective particles of the microcapsules do not adhere to each other. Preferably, microcapsules are heated to dry at a temperature ranging from a glass transition temperature of a lactic acid-glycolic acid polymer to a temperature about 30°C higher than the glass transition temperature. Here, a glass transition temperature is an intermediate point obtained by raising a temperature at a rate of 10 to 20°C per minute using a differential scanning calorimeter.

Anticoagulants may be added to the thus obtained microcapsules in order to prevent aggregation of each particles. As the anticoagulants, for example, water soluble polysaccharides such as mannitol, lactose, glucose, starches (e.g., corn starch and the like), hyaluronic acid or alkali metal salts thereof and the like, proteins such as glycine, fibrin, collagen and the like, inorganic salts such as sodium chloride, sodium hydrogen phosphate and the like are appropriately used.

Alternatively, microcapsules may be cooled after warming, to mold into a disc, a film, a bar (a rod) or the like as in the case of the above B-1-a).

In the aforementioned various processes, zinc oxide may be added to the organic solvent upon dissolution of a lactic acid-glycolic acid polymer in an organic solvent.

The amount of zinc oxide to be used is, for example, about 0.1 to about 100 parts by weight, preferably about 1 to about 20 parts by weight relative to 1 part by weight of a lactic acid-glycolic acid polymer.

In addition, a particle diameter of zinc oxide is usually about 0.001 to about 10 µm, preferably about 0.005 to about 1 µm.

A sustained release preparation obtained by using zinc oxide like this has excellent properties such as "a high drug entrapment ratio", "a small initial burst of a drug upon administration to the living body", "continuous release of a drug over a long period of time" and the like.

Upon production of a sustained release preparation of the present invention, BTC protein or a mutein thereof or a salt thereof is dissolved in an aqueous solution of ammonium acetate, which is lyophilized for use.

A BTC protein or a mutein thereof or a salt thereof obtained by treating with ammonium acetate like this has a small particle diameter and the excellent operability and, therefore, it is advantageous upon production of a sustained release preparation.

The thus obtained sustained release preparation of the present invention can be administered as it is or, optionally, by preparing into various dosage forms using a pharmaceutically acceptable additive (e.g., a stabilizing agent, a preservative, a soothing agent or the like). Examples of these preparations include a parenteral preparations (e.g., injections, implants, suppositories and the like), oral preparations (e.g., solid preparations such as capsules, tablets, granules, powders and the like, liquid preparations such as syrups, emulsions, suspensions and the like). Examples of the stabilizing agent as a pharmaceutically acceptable additive include human serum albumin, polyethylene glycol and the like. Example of the preservative include benzyl alcohol, phenol and the like. Examples of the soothing agent include benzalkonium chloride, procaine hydrochloride and the like. The amount of BTC protein or a mutein thereof or a salt thereof contained in the preparation of the present invention can be appropriately selected from the range of about 0.01 to about 100% (w/w) relative to the entire preparation.

A sustained release preparation of the present invention may be also produced by dissolving, suspending or emulsifying BTC protein or a mutein thereof or a salt thereof in an aqueous solvent (e.g., distilled water, physiological saline, Ringer's solution and the like) or an oily solvent (e.g., vegetable oils such as olive oil, sesame oil, cotton seed oil, corn oil and the like; propylene glycol and the like) and, sealing in a commercially available container for a sustained release preparation [e.g., Dulos (trade name, manufactured by Arza)].

A preparation of the present invention may be administered orally or parenterally but a parenteral preparation is preferable. Especially, an agent for systemic administration such as an agent for subcutaneous administration, an agent for intraperitoneal administration, and an agent for intramuscular administration, or an agent for local administration to the pancreas and the like are preferable. In particular, an agent for local administration to the pancreas is preferable.

In the present invention, an agent for local administration to the pancreas comprising BTC protein or a mutein thereof or a salt thereof may be a preparation comprising BTC protein or a mutein thereof or a salt thereof as it is or which is prepared into a variety of dosage forms optionally using a pharmaceutically acceptable carrier according to known pharmaceutical preparation production methods, and a sustained release preparation comprising BTC protein or a mutein thereof or a salt thereof is preferable.

Specifically, an agent for local administration to the pancreas is used by a method of administering a BTC sustained release preparation directly to the pancreas or its surroundings using by utilizing endoscope operation or the like, a method of administering a preparation to a pancreatic governing artery using a stent technique, a method of administering a preparation to the pancreas via a pancreatic duct using an oral endoscope and the like.

Examples of the dosage forms of a preparation of the present invention include a molded disc, a molded film, a molded bar, microcapsules and the like which are exemplified in the explanation for a method of producing the aforementioned BTC protein-comprising sustained release preparation. In order to supply BTC protein or a mutein thereof or a salt thereof to the pancreas locally and over a long period of time, a molded disc, a molded film and molded bar are preferable. Further, a molded bar is particularly preferable since the step of preparing this preparation is simple.

Since a preparation of the present invention is low in toxicity, it can be used as a safe preparation against pancreatic function disorder and the pancreatic function reduction of a mammal (e.g., human beings, monkeys, sacred baboons and chimpanzees, pigs, cows, sheep, horses, mice, rats and the like). Specifically, a preparation of the present invention has the activity of promoting differentiation of pancreatic beta cells as a cell differentiation promoter. That is, a preparation of the present invention can act on undifferentiated pancreatic stem cells to differentiate them into pancreatic beta cells. Pancreatic beta cells resulting from such differentiation and induction can secrete and produce insulin. In addition, a preparation of the present invention has an activity to differentiate and induce undifferentiated pancreatic stem cells to other cells of pancreas such as F cells producing pancreatic peptides (hereinafter abbreviated as PP in some cases). Further, a preparation of the present invention has an activity to improve glucose tolerance in vivo. Accordingly, a preparation of the present invention can be effectively used for treating or preventing diseases such as diabetes (e.g., insulin dependent diabetes), pancreatic function disorders in diabetes, pancreatic function reduction accompanied with senile insulin secretion reduction, diabetic complications such as neuropathy, nephropathy, retinopathy, cataract, macroangiopathy, osteopenia and the like), undifferentiated type pancreatic cancer and the like.

The dose of a preparation of the present invention can be appropriately selected depending upon the kind of contents of BTC protein or a mutein thereof or a salt thereof as an active ingredient, the dosage form, the duration time, the administration subject, the administration route, the object of administration, the target disease, clinical conditions and the like. For example, when a preparation is administered as an injection (e.g., a sustained release preparation of about 2 to about 3 months) for treating a diabetic adult patient (body weight about 60 kg), the dose is about 30 to about 600 mg/kg body weight as BTC protein or a mutein thereof or a salt thereof per one time.

Further, a preparation of the present invention can be used in combination with a drug such as a therapeutic agent for diabetes mellitus, a therapeutic agent for diabetic complications, an antihyperlipidemic agent, a hypotensive agent, an antiobesity agent, a diuretic agent, a chemotherapeutic agent, an immunotherapeutic agent and the like (hereinafter, abbreviated as a concomitant drug). On such occasions, the time of administration of the preparation of the present invention and that of the concomitant drug is not limited. They may be administered simultaneously or at staggered times to the administration subject. The dose of the concomitant drug can be appropriately selected based on the dose which is clinically employed. The proportion of the preparation of the present invention and the concomitant drug can be appropriately selected according to the administration subject, administration route, target disease, clinical condition, combination, and the like.

Examples of the therapeutic agent for diabetes mellitus include insulin preparations (e.g., animal insulin preparations extracted from the bovine or swine pancreas; human insulin preparations synthesized by a genetic engineering technique using Escherichia coli or a yeast), insulin sensitizers (e.g., pioglitazone hydrochloride, troglitazone, rosiglitazone (or its maleate), JTT-501, MCC-555, R-119702), α-glucosidase inhibitors (e.g., voglibose, acarbose, miglitol), biguanides (e.g., phenformin, metformin, buformin), or sulfonylureas (e.g., tolbutamide, glibenclamide, gliclazide, chlorpropamide, tolazamide, acetohexamide, glyclopyramide, glimepiride) and other insulin secretagogues (e.g., repaglinide, senaglinide, mitiglinide, GLP-1).

Examples of the therapeutic agent for diabetic complications include aldose reductase inhibitors (e.g., tolrestat, epalrestat, zenarestat, SNK-860, CT-112), neurotrophic factors (e.g., NGF, NT-3), active oxygen scavengers (e.g. thioctic acid), cerebral vasodilators (e.g., tiapuride, mexiletine).

Examples of the antihyperlipidemic agent include statin compounds which are cholesterol synthesis inhibitors (e.g., cerivastatin, pravastatin, simvastatin, lovastatin, atorvastatin, fluvastatin), squalene synthase inhibitors or fibrate compounds (e.g., bezafibrate, clofibrate, simfibrate, clinofibrate) having a triglyceride lowering action.

Examples of the hypotensive agent include angiotensin converting enzyme inhibitors (e.g., captopril, enalapril, delapril), angiotensin II antagonists (e.g., candesartan cilexetil, losartan), calcium antagonist (e.g., nicardipine, nifedipine, diltiazem, manidipine).

Examples of the antiobesity agent include antiobesity drugs acting on the central nervous system (e.g. dexfenfluramine, fenfluramine, phentermine, sibutramine, anfepramon, dexamphetamine, mazindol, phenylpropanolamine, clobenzorex), pancreatic lipase inhibitors (e.g. orlistat), β3 agonists (e.g. CL-316243, SR-58611-A, UL-TG-307, AJ-9677), anorectic peptides (e.g. leptin), cholecystokinin agonists (e.g. lintitript, FPL-15849).

Examples of the diuretic agent include xanthine derivatives (e.g., theobromine and sodium salicylate, theobromine and calcium salicylate), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlorthalidone, mefruside, indapamide), azosemide, isosorbide, ethacrynic acid, piretanide, bumetanide, furosemide.

Examples of the chemotherapeutic agent include alkylating agents (e.g., cyclophosphamide, ifosamide), metabolic antagonists (e.g., methotrexate, 5-fluorouracil), antitumor antibiotics (e.g., mitomycin, adriamycin), plant-derived antitumor agents (e.g., vincristine, vindesine, Taxol), cisplatin, carboplatin, etoposide. Among these, 5-fluorouracil derivatives such as Furtulon and Neo-Furtulon are preferable.

Examples of the immunotherapeutic agent include microorganism- or bacterium-derived components (e.g., muramyl dipeptide derivatives, Picibanil), immunopotentiator polysaccharides (e.g., lentinan, schizophyllan, krestin), genetically engineered cytokines (e.g., interferons, interleukins (IL)), colony stimulating agents (e.g., granulocyte colony stimulating factor, erythropoietin), etc. Among these, IL-1, IL-2, IL-12 and the like are preferable.

Further, agents whose effects of ameliorating cachexia have been confirmed in animal models or clinically, namely cyclooxygenase inhibitors (e.g., indomethacin) (Cancer Research, vol. 49, pp. 5935-5939, 1989), progesterone derivatives (e.g., megestrol acetate) (Journal of Clinical Oncology, vol. 12, pp. 213-225, 1994), glucocorticoids (e.g. dexamethasone), metoclopramide pharmaceuticals, tetrahydrocannabinol pharmaceuticals (the above references are applied to both), fat metabolism ameliorating agents (e.g., eicosapentanoic acid) (British Journal of Cancer, vol. 68, pp. 314-318, 1993), growth hormones, IGF-1, and antibodies to the cachexia-inducing factor TNF-α, LIF, IL-6 or oncostatin M, can also be used in combination with the preparation of the present invention.

The following Examples and Test Examples illustrate the present invention in more detail but the present invention is not limited to them.

When a base, an amino acid and the like are denoted by abbreviation, they are based on IUPAC-IUB Commission on Biochemical Nomenclature abbreviation or the conventional abbreviation in the art, and embodiments thereof are shown below. In addition, when there may be an optical isomer regarding an amino acid, an amino acid represents L-amino acid.
- DNA:: deoxyribonucleic acid
- cDNA:: complementary deoxyribonucleic acid
- A:: adenine
- T:: thymine
- G:: guanine
- C:: cytosine
- Gly:: glycine
- Ala:: alanine
- Val:: valine
- Leu:: leusine
- Ile:: isoleusine
- Ser:: serine
- Thr:: threonine
- Cys:: cysteine
- Met:: methionine
- Glu:: glutamic acid
- Asp:: aspartic acid
- Lys:: lysine
- Arg:: arginine
- His:: histidine
- Phe:: phenylalanine
- Tyr:: thyrosine
- Trp:: tryptophan
- Pro:: proline
- Asn:: asparagine
- Gln:: glutamine

SEQ ID Nos. in Sequence Listing in the present specification denote the following sequences:

### [SEQ ID No.:1]

Denotes an amino acid sequence of a human-derived BTC protein.

### [SEQ ID No.:2]

Denotes an amino acid sequence of a mouse-derived BTC protein.

### [SEQ ID No.:3]

Denotes a partial amino acid sequence of 36th to 47th amino acids of an amino acid sequence represented by SEQ ID No.:1 or SEQ ID No.:2.

### [SEQ ID No.:4]

Denotes a partial amino acid sequence of 49th to 55th amino acids of an amino acid sequence represented by SEQ ID No.:1 or SEQ ID No.:2.

### [SEQ ID No.:5]

Denotes a partial amino acid sequence of 57th to 62nd amino acids of an amino acid sequence represented by SEQ ID No.:1 or SEQ ID No.:2.

### [SEQ ID No.:6]

Denotes a partial amino acid sequence of 70th to 80th amino acids of an amino acid sequence represented by SEQ ID No.:1 or SEQ ID No.:2.

### [SEQ ID No.:7]

Denotes a base sequence of a DNA encoding a protein having an amino acid sequence of a human-derived BTC protein represented by SEQ ID No.:1.

### [SEQ ID No.:8]

Denotes a base sequence of a DNA encoding a protein having an amino acid sequence of a mouse-derived BTC protein represented by SEQ ID No.:2.

### Example 1

After 760 mg of TetraGlycerol MonoPalmitate (TGMP) which is one of polyglycerin fatty acid esters was heated to melt at 60 to 75°C, 20 mg of lyophilized powder of rhBTC (recombinant human betacellulin protein) was added, which was stirred and admixed, then about 30 mg was aspirated with a 14G indwelling needle and cooled to solidify at room temperature. In order to take out the solidified rhBTC-containing TGMP, the dwelling needle was heated at 55°C for about one minute to perform extrusion-molding. The resulting cyrindrical pellet was cut into a length of 20 mm to obtain a sustained release rhBTC-containing TGMP preparation having a diameter of about 1.2 mm and a length of about 20 mm. The content of a drug (rhBTC) in a preparation was 2.5% as determined as a blending amount.

### Example 2

After 760 mg of TetraGlycerol DiPalmitate (TGDP) which is one of polyglycerin fatty acid esters was heated to melt at 60 to 75°C, 20 mg of lyophilized powder of rhBTC was added, which was stirred and admixed, then about 30 mg was aspirated with a 14G indwelling needle and cooled to solidify at room temperature. In order to take out the solidified rhBTC-containing TGDP, the dwelling needle was heated at 55°C for about one minute to perform extrusion-molding. The resulting cyrindrical pellet was cut into a length of 20 mm to obtain a sustained release rhBTC-containing TGDP preparation having a diameter of about 1.2 mm and a length of about 20 mm. The content of a drug in a preparation was 2.5% as determined as a blending amount.

### Example 3

After 760 mg of TetraGlycerol TriPalmitate (TGTP) which is one of polyglycerin fatty acid esters was heated to melt at 60 to 75°C, 20 mg of lyophilized powder of rhBTC was added, which was stirred and admixed, then about 30 mg was aspirated with a 14G indwelling needle and cooled to solidify at room temperature. In order to take out the solidified rhBTC-containing TGTP, the dwelling needle was heated at 55°C for about one minute to perform extrusion-molding. The resulting cyrindrical pellet was cut into a length of 20 mm to obtain a sustained release rhBTC-containing TGTP preparation having a diameter of about 1.2 mm and a length of about 20 mm. The content of a drug in a preparation was 2.5% as determined as a blending amount.

### Example 4

After 760 mg of TetraGlycerol HexaPalmitate (TGHP) which is one of polyglycerin fatty acid esters was heated to melt at 60 to 75°C, 20 mg of lyophilized powder of rhBTC was added, which was stirred and admixed, then about 30 mg was aspirated with a 14G indwelling needle and cooled to solidify at room temperature. In order to take out the solidified rhBTC-containing TGHP, the dwelling needle was heated at 55°C for about one minute to perform extrusion-molding. The resulting cyrindrical pellet was cut into a length of 20 mm to obtain a sustained release rhBTC-containing TGHP preparation having a diameter of about 1.2 mm and a length of about 20 mm. The content of a drug in a preparation was 2.5% as determined as a blending amount.

### Example 5

After 760 mg of TetraGlycerol MonoStearate (TGMS) which is one of polyglycerin fatty acid esters was heated to melt at 60 to 75°C, 20 mg of lyophilized powder of rhBTC was added, which was stirred and admixed, then about 30 mg was aspirated with a 14G indwelling needle and cooled to solidify at room temperature. In order to take out the solidified rhBTC-containing TGMS, the dwelling needle was heated at 55°C for about one minute to perform extrusion-molding. The resulting cyrindrical pellet was cut into a length of 20 mm to obtain a sustained release rhBTC-containing TGMS preparation having a diameter of about 1.2 mm and a length of about 20 mm. The content of a drug in a preparation was 2.5% as determined as a blending amount.

### Example 6

After 760 mg of TetraGlycerol DiStearate (TGDS) which is one of polyglycerin fatty acid esters was heated to melt at 60 to 75°C, 20 mg of lyophilized powder of rhBTC was added , which was stirred and admixed, then about 30 mg was aspirated with a 14G indwelling needle and cooled to solidify at room temperature. In order to take out the solidified rhBTC-containing TGDS, the dwelling needle was heated at 55°C for about one minute to perform extrusion-molding. The resulting cyrindrical pellet was cut into a length of 20 mm to obtain a sustained release rhBTC-containing TGDS preparation having a diameter of about 1.2 mm and a length of about 20 mm. The content of a drug in a preparation was 2.5% as determined as a blending amount.

### Example 7

After 760 mg of TetraGlycerol TriStearate (TGTS) which is one of polyglycerin fatty acid esters was heated to melt at 60 to 75°C, 20 mg of lyophilized powder of rhBTC was added , which was stirred and admixed, then about 30 mg was aspirated with a 14G indwelling needle and cooled to solidify at room temperature. In order to take out the solidified rhBTC-containing TGTS, the dwelling needle was heated at 55°C for about one minute to perform extrusion-molding. The resulting cyrindrical pellet was cut into a length of 20 mm to obtain a sustained release rhBTC-containing TGTS preparation having a diameter of about 1.2 mm and a length of about 20 mm. The content of a drug in a preparation was 2.5% as determined as a blending amount.

### Example 8

After 760 mg of TetraGlycerol HexaStearate (TGHS) which is one of polyglycerin fatty acid esters was heated to melt at 60 to 75°C, 20 mg of lyophilized powder of rhBTC was added, which was stirred and admixed, then about 30 mg was aspirated with a 14G indwelling needle and cooled to solidify at room temperature. In order to take out the solidified rhBTC-containing TGHS, the dwelling needle was heated at 55°C for about one minute to perform extrusion-molding. The resulting cyrindrical pellet was cut into a length of 20 mm to obtain a sustained release rhBTC-containing TGHS preparation having a diameter of about 1.2 mm and a length of about 20 mm. The content of a drug in a preparation was 2.5% as determined as a blending amount.

### Example 9

After 760 mg of HexaGlycerol PentaStearate (HGPS) which is one of polyglycerin fatty acid esters was heated to melt at 60 to 75°C, 20 mg of lyophilized powder of rhBTC was added, which was stirred and admixed, then about 30 mg was aspirated with a 14G indwelling needle and cooled to solidify at room temperature. In order to take out the solidified rhBTC-containing HGPS, the dwelling needle was heated at 55°C for about one minute to perform extrusion-molding. The resulting cyrindrical pellet was cut into a length of 20 mm to obtain a sustained release rhBTC-containing HGPS preparation having a diameter of about 1.2 mm and a length of about 20 mm. The content of a drug in a preparation was 2.5% as determined as a blending amount.

### Example 10

After 1560 mg of TetraGlycerol HexaPalmitate (TGHP) which is one of polyglycerin fatty acid esters was heated to melt at 70°C, 40 mg of lyophilized powder of rhBTC was added, which was stirred and admixed. The mixture was aspirated with a transplantation needle having an inner diameter of 1.5 mm and cooled to solidify at room temperature. In order to take out the solidified rhBTC-containing TGHP, the transplantation needle was heated at 60°C for 1 to 3 minutes to perform extrusion-molding. The resulting cyrindrical pellet was cut into a length of 10 mm to obtain a sustained release rhBTC-containing TGHP preparation having a diameter of 1.5 mm and a length of 10 mm. The content of a drug in a preparation was 2.5% as determined as a blending amount.

### Example 11

After 1560 mg of TetraGlycerol HexaPalmitate (TGHP) which is one of polyglycerin fatty acid esters was heated to melt at 70°C, 40 mg of lyophilized powder of rhBTC was added, which was stirred and admixed. The mixture was aspirated with a transplantation needle having an inner diameter of 2.0 mm and cooled to solidify at room temperature. In order to take out the solidified rhBTC-containing TGHP, the transplantation needle was heated at 60°C for 1 to 3 minutes to perform extrusion-molding. The resulting cyrindrical pellet was cut into a length of 10 mm to obtain a sustained release rhBTC-containing TGHP preparation having a diameter of 2.0 mm and a length of 10 mm. The content of a drug in a preparation was 2.5% as determined as a blending amount.

### Example 12

After 1560 mg of HexaGlycerol PentaStearate (HGPS) which is one of polyglycerin fatty acid esters was heated to melt at 70°C, 40 mg of lyophilized powder of rhBTC was added, which was stirred and admixed. The mixture was aspirated with a transplantation needle having an inner diameter of 1.5 mm and cooled to solidify at room temperature. In order to take out the solidified rhBTC-containing HGPS, the transplantation needle was heated at 60°C for 1 to 3 minutes to perform extrusion-molding. The resulting cyrindrical pellet was cut into a length of 10 mm to obtain a sustained release rhBTC-containing HGPS preparation having a diameter of 1.5 mm and a length of 10 mm. The content of a drug in a preparation was 2.5% as determined as a blending amount.

### Example 13

After 1560 mg of HexaGlycerol PentaStearate (HGPS) which is one of polyglycerin fatty acid esters was heated to melt at 70°C, 40 mg of lyophilized powder of rhBTC was added, which was stirred and admixed. The mixture was aspirated with a transplantation needle having an inner diameter of 2.0 mm and cooled to solidify at room temperature. In order to take out the solidified rhBTC-containing HGPS, the transplantation needle was heated at 60°C for 1 to 3 minutes to perform extrusion-molding. The resulting cyrindrical pellet was cut into a length of 10 mm to obtain a sustained release rhBTC-containing HGPS preparation having a diameter of 2.0 mm and a length of 10 mm. The content of a drug in a preparation was 2.5% as determined as a blending amount.

### Example 14

4 g of a lactic acid-glycolic acid polymer (PLGA) having a L/G ratio of 75/25 and a molecular weight of 12700 was dissolved in 3.3 ml of dichloromethane, and 0.8 ml of an aqueous solution of rhBTC having the concentration of 50 mg/ml was added under ice-cooling, which was emulsified with Polytron at 20000 rpm for 20 seconds. The emulsion was poured into 800 ml of a 0.1% aqueous PVA solution while stirring, and dried in water for 3 hours while stirring with a propeller. The dried material was classified with a 125 µm mesh, which was centrifuged, washed and lyophilized to obtain sustained release rhBTC-containing microcapsules having the drug content of 1%.

### Example 15

4 g of a lactic acid-glycolic acid polymer having a L/G ratio of 75/25 and a molecular weight of 12700 was dissolved in 3.3 ml of dichloromethane, and 0.8 ml of an aqueous solution of rhBTC having the concentration of 150 mg/ml was added under ice-cooling, which was emulsified with Polytron at 20000 rpm for 20 seconds. The emulsion was poured into 800 ml of a 0.1% aqueous PVA solution while stirring, and dried in water for 3 hours while stirring with a propeller. The dried material was classified with a 125 µm mesh, which was centrifuged, washed and lyophilized to obtain sustained release rhBTC-containing microcapsules having the drug content of 3%.

### Example 16

4 g of a lactic acid-glycolic acid polymer having a L/G ratio of 75/25 and a molecular weight of 12700 was dissolved in 3.3 ml of dichloromethane, and 0.8 ml of an aqueous solution of rhBTC having the concentration of 250 mg/ml was added under ice-cooling, which was emulsified with Polytron at 20000 rpm for 20 seconds. The emulsion was poured into 800 ml of a 0.1% aqueous PVA solution while stirring, and dried in water for 3 hours while stirring with a propeller. The dried material was classified with a 125 µm mesh, which was centrifuged, washed and lyophilized to obtain sustained release rhBTC-containing microcapsules having the drug content of 5%.

### Example 17

10 g of a lactic acid-glycolic acid polymer (polylactic acid) having a L/G ratio of 100/0 and a molecular weight of 18000 was dissolved in 8.3 ml of dichloromethane, and 2.08 ml of an aqueous solution of rhBTC having the concentration of 273 mg/ml was added under ice-cooling, which was emulsified with Polytron at 24000 rpm for 30 seconds. The emulsion was poured into 1600 ml of a 0.1% aqueous PVA solution while stirring, and dried in water for 2 hours while stirring with a propeller. The dried material was classified with a 125 µm mesh, which was centrifuged, washed and lyophilized to obtain sustained release rhBTC-containing microcapsules having the drug content of 5.2%.

### Example 18

6.7 g of a lactic acid-glycolic acid polymer (polylactic acid) having a L/G ratio of 100/0 and a molecular weight of 18000 was dissolved in 6.7 ml of dichloromethane, and 1.67 ml of an aqueous solution of rhBTC having the concentration of 265 mg/ml was added under ice-cooling, which was emulsified with Polytron at 24000 rpm for 30 seconds. The emulsion was dried under vacuum overnight to obtain a rhBTC-containing PLA powder. This was filled into a Teflon tube having an inner diameter of 2.0 mm and heated at 60°C for 15 minutes. After heating, the powder was compressed with a bar, and cooled and molded. A bar-like preparation having a longitudinal diameter of 2.0 mm and a length of 1 cm was obtained.

### Example 19

8.0 g of polylactic acid (PLA) having a molecular weight of 18000 was dissolved in 6.7 ml of dichloromethane, and 1.67 ml of an aqueous solution of rhBTC having the concentration of 264.72 mg/ml was added, which was emulsified with Polytron at 24000 rpm for 30 seconds. The emulsion was dried overnight under vacuum to obtain 8.09 g of a rhBTC/PLA powder. 46 µg of this powder was filled into a Teflon tube having an inner diameter of 2.0 mm and heated at 60°C for 15 minutes. After heating, the powder was compressed with a bar having a longitudinal diameter of 2.0 mm, and cooled and molded. A rod-like preparation containing 5.16 % of rhBTC was obtained.

### Example 20

7.0 g of polylactic acid (PLA) having a molecular weight of 12000 was dissolved in 5.8 ml of dichloromethane, and 1.46 ml of an aqueous solution of rhBTC having the concentration of 368 mg/ml ml was added, which was emulsified with Polytron at 25000 rpm for 30 seconds. The emulsion was poured into 1170 ml of a 0.1% aqueous PVA solution while stirring with a homomixer (7000 rpm), and dried in water for 2 hours while stirring with a propeller stirrer. After the dried material was classified with a 125 µm mesh, centrifugation and washing were conducted, then lyophilization was conducted to obtain microcapsules having the drug content of 5.75%. The microcapsules were filled into a Teflon tube having an inner diameter of 2.0 mm, and heated at 60°C for 15 minutes. After heating, the material was compressed with a bar having a longitudinal diameter of 2.0 mm, cooled and molded. A rod-like preparation containing 5.75% rhBTC was obtained.

### Example 21

7.0 g of polylactic acid (PLA) having a molecular weight of 12000 and 33.6 mg of zinc oxide were dissolved in 5.8 ml of dichloromethane, and 1.41 ml of an aqueous solution of rhBTC having the concentration of 368 mg/ml was added, which was emulsified with Polytron at 25000 rpm for 30 seconds. The emulsion was poured into 1170 ml of a 0.1% aqueous PVA solution while stirring with a homomixer (7000 rpm), and dried in water for 2 hours while stirring with a propeller stirrer. The dried material was classified with a 125 µm mesh, which was centrifuged, washed and lyophilized to obtain microcapsules having the drug content of 6.55%. The microcapsules were filled into a Teflon tube having an inner diameter of 2.0 mm, and heated at 60°C for 15 minutes. After heating, the material was compressed with a bar having a longitudinal diameter of 2.0 mm, cooled and molded. A rod-like preparation containing 6.55% rhBTC was obtained. Example 22

2.4 g of a lactic acid-glycolic acid polymer (PLGA) having a L/G ratio of 75/25 and a molecular weight of 12700 was dissolved in 2.0 ml of dichloromethane, and 1.58 ml of an aqueous solution of rhBTC having the concentration of 439.03 mg/ml was added , which was emulsified with Polytron at 25000 rpm for 30 seconds. The emulsion was poured into 400 ml of 0.1% aqueous PVA solution while stirring with a homomixer (7000 rpm) and dried in water for 2 hours while stirring with a propeller stirrer. The dried material was classified with a 125 µm mesh, centrifuged, washed and lyophilized to obtain microcapsules. The microcapsules were filled into a Teflon tube having an inner diameter of 2.0 mm and heated at 60°C for 15 minutes. After heating, the material was compressed with a bar having a longitudinal diameter of 2.0 mm, and cooled and molded. A rod-like preparation containing 10.46% rhBTC was obtained.

### Example 23

3.5 g of a lactic acid-glycolic acid polymer (PLGA) having a L/G ratio of 75/25 and a molecular weight of 12700 was dissolved in dichloromethane, and 384 mg of rhBTC bulk powder was added and dispersed with Polytron (20000 rpm, 30 seconds) to obtain a s/o dispersion. This was dried under vacuum overnight, filled into a Teflon tube having an inner diameter of 2.0 mm, and heated at 60°C for 15 minutes. After heating, the material was compressed with a bar having a longitudinal diameter of 2.0 mm, cooled and molded. A rod-like preparation containing 8.14% rhBTC was obtained.

### Example 24

2.4 g of a lactic acid-glycolic acid polymer (PLGA) having a L/G ratio of 75/25 and a molecular weight of 12700 and 24.03 mg of zinc oxide were dissolved in 2.0 ml of dichloromethane. To the resulting solution was added a solution of 282.87 mg of rhBTC dissolved in 500 mg of distilled water, which was emulsified with Polytron at 25000 rpm for 30 seconds. The emulsion was poured into 400 ml of 0.1% aqueous PVA solution while stirring with a homomixer (7000 rpm), and dried in water for 2 hours while stirring with a propeller stirrer. The dried material was classified with a 125 µm mesh, centrifuged, washed and lyophilized to obtain microcapsules. The microcapsules were filled into a Teflon tube having an inner diameter of 2.0 mm, and heated at 60°C for 15 minutes. After heating, the material was compressed with a bar having a longitudinal diameter of 2.0 mm, cooled and molded. A rod-like preparation containing 9.19% rhBTC was obtained.

### Example 25

2.4 g of a lactic acid-glycolic acid polymer (PLGA) having a L/G ratio of 75/25 and a molecular weight of 12700 and 23.95 mg of zinc oxide were dissolved in 2.0 ml of dichloromethane, and 269.72 ml of rhBTC was added, which was emulsified with Polytron at 25000 rpm for 30 seconds. The emulsion was poured into 1170 ml of 0.1% aqueous PVA solution while stirring with a homomixer (7000 rpm), and dried in water for 2 hours while stirring with a propeller stirrer. The dried material was classified with a 125 µm mesh, centrifuged, washed and lyophilized to obtain microcapsules having the drug content of 6.55%. The microcapsules were filled into a Teflon tube having an inner diameter of 2.0 mm, and heated at 60°C for 15 minutes. After heating, the material was compressed with a bar having a longitudinal diameter of 2.0 mm, cooled and molded. A rod-like preparation containing 8.72% rhBTC was obtained.

### Example 26

rhBTC bulk was dissolved in a 4.39 mM aqueous ammonium acetate solution to 2 mg/ml, which was rapidly frozen and dried under vacuum. 3.5 g of polylactic acid (PLA) having a molecular weight of 12000 and 35.5 mg of zinc oxide were dissolved in 3.5 ml of dichloromethane, and to this was added 399 mg of rapidly lyophilized rhBTC, which was dispersed with Polytron. The resulting s/o dispersion system was dried under vacuum overnight, filled into a Teflon tube having an inner diameter of 2.0 mm, and heated at 60°C for 15 minutes. After heating, the material was compressed with a bar having a longitudinal diameter of 2.0 mm, cooled and molded. A rod-like preparation containing 10.11% rhBTC was obtained.

### Example 27

rhBTC bulk was dissolved in a 4.39 mM aqueous ammonium acetate solution to 2 mg/ml, which was rapidly frozen and dried under vacuum. 4.5 g of a lactic acid-glycolic acid polymer (PLGA) having a L/G ratio of 75/25 and a molecular weight of 12700 and 45.5 mg of zinc oxide were dissolved in 4.5 ml of dichloromethane, and to this was added 515 mg of rapidly lyophilized rhBTC, which was dispersed with Polytron. The resulting s/o dispersion system was dried under vacuum over night, filled into a Teflon tube having an inner diameter of 2.0 mm and heated at 60°C for 15 minutes. After heating, the material was compressed with a bar having a longitudinal diameter of 2.0 mm, cooled and molded. A rod-preparation containing 10.02% of rhBTC was obtained.

### Test Example 1

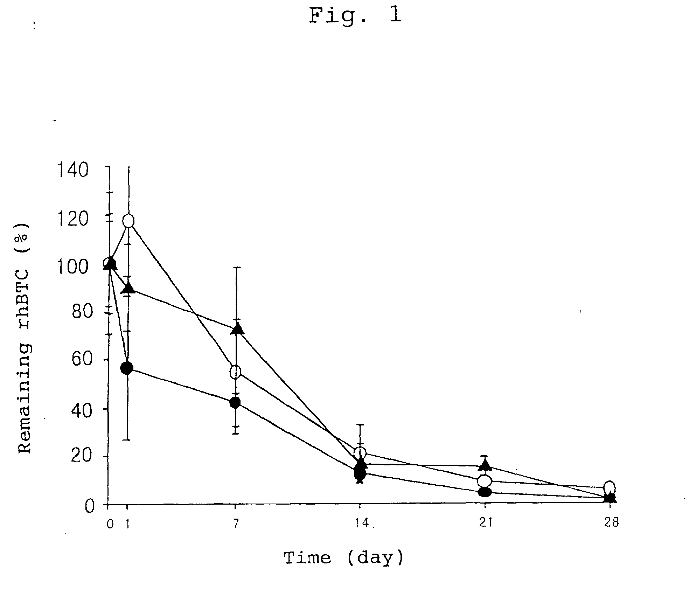

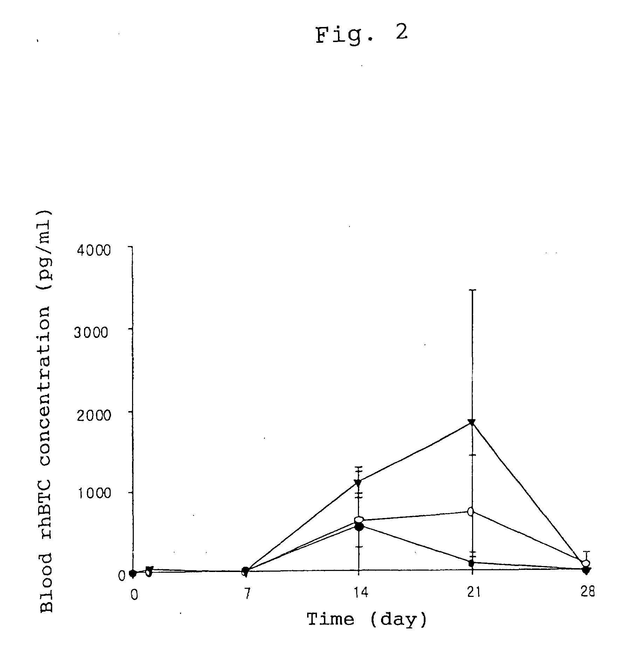

Sustained release PLGA microcapsules containing rhBTC prepared in Examples 14, 15 and 16 were administered to rats subcutaneously so that a rhBTC amount was 3.2 mg/kg, 5.9 mg/kg and 10.0 mg/kg, respectively, and a change with time of the remaining rhBTC at an administered site and the blood rhBTC concentration were measured over one month. In respective preparations, rhBTC remained over one month as shown in Fig. 1 and the blood rhBTC concentration was detected over one month as shown in Fig. 2. Thereby, it is apparent that a sustained release preparation of the present invention has an excellent sustained-release ability.

### Test Example 2

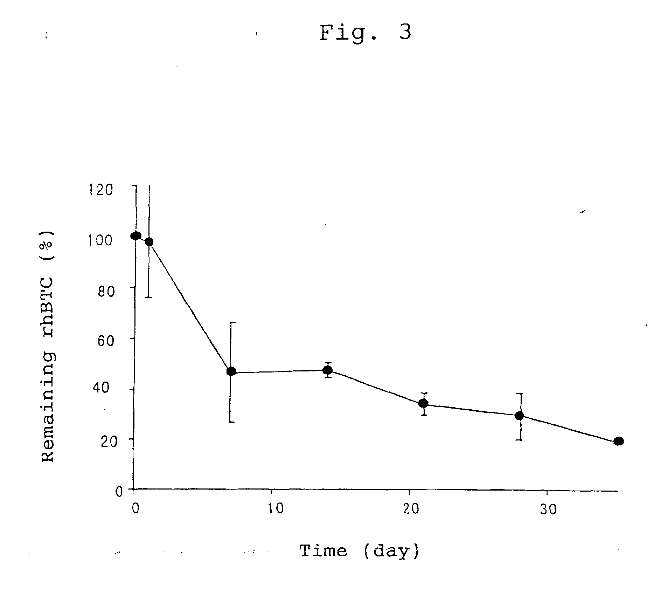

The sustained release HGPS preparation containing rhBTC prepared in Example 13 was administered to rats subcutaneously so that a rhBTC amount was 4.1 mg/kg, and a change with time of the remaining rhBTC was measured over one month. As shown in Fig. 3, rhBTC remained over one month. Thereby, it is apparent that a sustained release preparation of the present invention has an excellent sustained-release ability.

### Test Example 3

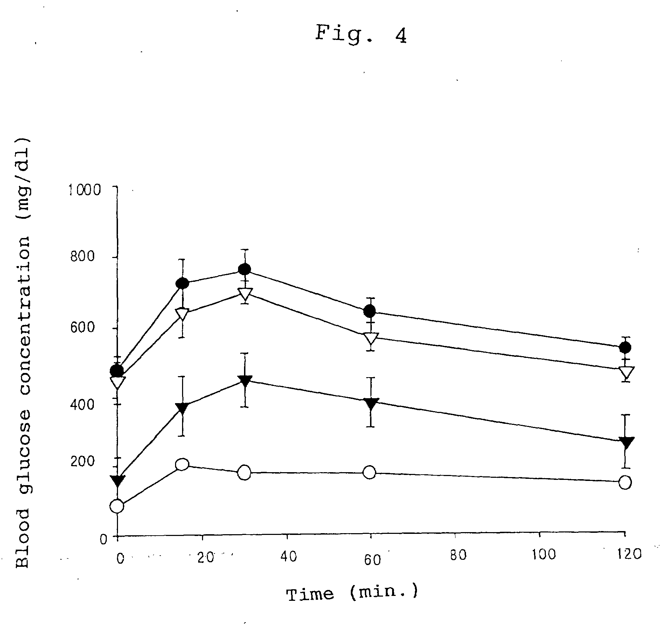

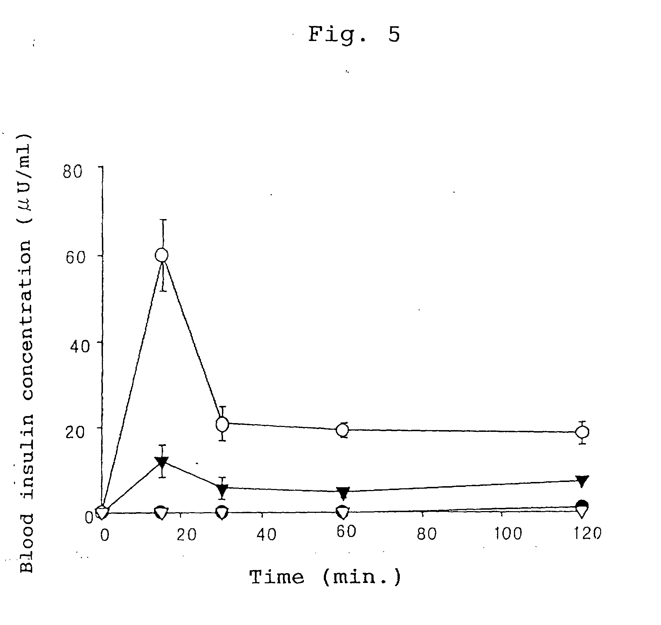

The sustained release HGPS preparation containing rhBTC which was prepared in Example 13 and whose sustained-release ability was confirmed in Test Example 2, was administered to STZ diabetic model rats subcutaneously or pancreas-locally so that a rhBTC amount was 45 mg/kg, and the blood glucose concentration and the blood insulin concentration were measured after 8 weeks. As shown in Fig. 4, a hypoglycemic action (decrease in the blood glucose concentration) was observed in the subcutaneously administered group and the pancreas-locally administered group as compared with a non-treated diabetic rat group, and the glucose tolerance was improved. In addition, as shown in Fig. 5, in a pancreas-locally administered group, secretion of insulin was confirmed. Thereby, it is apparent that a preparation of the present invention improves the pancreatic function and is useful for treating or preventing diabetes.

### Industrial Applicability

A preparation of the present invention comprising BTC protein or a mutein thereof or a salt thereof improves pancreatic function, and is useful for treating or preventing diabetes and the like.

## Claims

1. A sustained release preparation comprising a betacellulin protein or a mutein thereof or a salt thereof.

2. The sustained release preparation according to claim 1, wherein the betacellulin protein is a protein having the amino acid sequence represented by at least one SEQ ID No. selected from the group consisting of SEQ ID No.:3, SEQ ID No.:4, SEQ ID No.:5 and SEQ ID No.:6.

3. The sustained release preparation according to claim 1, wherein the betacellulin protein is a protein having the amino acid sequence represented by SEQ ID No.:1 or a protein having the amino acid sequence represented by SEQ ID No.:2.

4. The sustained release preparation according to claim 1, wherein the betacellulin protein is a protein having the amino acid sequence represented by SEQ ID No.:1.

5. The sustained release preparation according to claim 1, wherein the mutein of a betacellulin protein is a protein having ① the amino acid sequence in which about 1 to 40 amino acids of the amino acid sequence represented by SEQ ID No.:1 or SEQ ID No.:2 are missing, ② the amino acid sequence in which about 1 to 40 amino acids of the amino acid sequence represented by SEQ ID No.:1 or SEQ ID No.:2 are replaced by other amino acids, or ③ the amino acid sequence in which about 1 to 40 amino acids are added to the amino acid sequence represented by SEQ ID No.:1 or SEQ ID No.:2.

6. The sustained release preparation according to claim 1, wherein the mutein of a betacellulin protein is a protein having the amino acid sequence in which 12 or 30 amino acids are missing from an N terminal of the amino acid sequence represented by SEQ ID No.:1.

7. The sustained release preparation according to claim 1, which comprises a betacellulin protein or a mutein thereof or a salt thereof and a carrier.

8. The sustained release preparation according to claim 7, wherein the carrier is a polymer.

9. The sustained release preparation according to claim 8, wherein the polymer is a biodegradable polymer.

10. The sustained release preparation according to claim 9, wherein the biodegradable polymer is an aliphatic polyester.

11. The sustained release preparation according to claim 10, wherein the aliphatic polyester is lactic acid-glycolic acid polymer.

12. The sustained release preparation according to claim 11, wherein the lactic acid/glycolic acid composition ratio of the lactic acid-gycolic acid polymer is about 100/0 to about 40/60.

13. The sustained release preparation according to claim 11, wherein a weight average molecular weight of the lactic acid-glycolic acid polymer is about 3,000 to about 80,000.

14. The sustained release preparation according to claim 8, wherein the polymer is a non-biodegradable polymer.

15. The sustained release preparation according to claim 14, wherein the non-biodegradable polymer is a polyglycerin fatty acid ester.

16. The sustained release preparation according to claim 1, which is a pancreatic function improving agent.

17. The sustained release preparation according to claim 1, which is an agent for treating or preventing diabetes.

18. The sustained release preparation according to claim 1, which is an agent for parenteral administration.

19. The sustained release preparation according to claim 1, which is an agent for local administration to the pancreas.

20. The sustained release preparation according to claim 1, which is an agent for subcutaneous administration.

21. The sustained release preparation according to claim 1, which is an agent for intramuscular administration.

22. The sustained release preparation according to claim 1, which is an agent for intraperitoneal administration.

23. An agent for local administration to the pancreas, which comprises a betacellulin protein or a mutein thereof or a salt thereof.

24. Use of a betacellulin protein or a mutein thereof or a salt thereof for the manufacture of a sustained release preparation.

25. Use of a betacellulin protein or a mutein thereof or a salt thereof for the manufacture of an agent for local administration to the pancreas.

26. A method for treating diabetes, which comprises administering an effective amount of a sustained release preparation as defined in claim 1 to a mammal.

27. A method for treating diabetes, which comprises locally administering an effective amount of an agent for local administration as defined in claim 23 to a mammal.
